# EUROPEAN PATENT APPLICATION

(11) **EP 4 468 271 A1**
(43) Date of publication of application: **27.11.2024**
(21) Application number: 24174828.4
(22) Date of filing: 08.05.2024
(51) Int. Cl.: G08G 1/00

(54) **MULTI-LEVEL WARNING BASED ON DRIVER SENTIMENT**

(30) Priority: 18.05.2023 US 202363467410 P
(71) Applicant: Cerence Operating Company, Burlington, MA 01803 (US)
(72) Inventor: KANE, Mark, Burlington, MA 01803 (US)
(74) Representative: Taruttis, Tilman

(57) **Abstract**

A method for providing a driver with a warning includes determining that a vehicle is being operated in a manner that fails to comply with a constraint imposed on motion of vehicles on a section of a road, determining that a driver of the vehicle exhibits a non-neutral sentiment, based on having determined that the driver exhibits a non-neutral sentiment, selecting a level of obtrusiveness for the warning message, and outputting the warning message at that level.

## Description

### Background

Different segments of a road often have different speed limits. A driver may not be aware of when a speed limit has changed. As a result, a driver may inadvertently drive at a speed higher than that permitted.

It is useful to provide a warning to the driver who is driving in a manner that does not comply with the speed limit. Known methods of providing such warnings rely on the speed of the vehicle and the speed limit at the vehicle's location.

### Summary

In one aspect, the invention features a method that includes determining that a vehicle is being operated in a manner that fails to comply with a constraint imposed on motion of vehicles on a section of a road, determining that a driver of the vehicle exhibits a non-neutral sentiment, based on having determined that the driver exhibits a non-neutral sentiment, selecting a level of obtrusiveness for the warning message, and outputting the warning message at that level.

Practices of the method include those in which the constraint is a speed limit.

Other practices of the method include those in which the level is selected based on an extent to which the non-neutral sentiment deviates from a neutral sentiment.

Among the practices of the method are those in which determining that a driver of the vehicle exhibits a non-neutral sentiment comprises making the determination based at least in part on a signal from a camera that is pointing at the driver, based at least in part on a signal from a microphone that is directed towards the driver, or based in part on both the signal from the camera and the signal from the microphone.

Still other practices include those in which determining the constraint is based at least in part on observations of signage by an external camera and those in which such a determination is based at least in part on a GPS signal.

In another aspect, the invention features a warning system for a vehicle. The warning system includes an escalator and a level selector. The level selector receives a constraint signal, which is indicative on a constraint on motion of a vehicle on a section of a road, a motion signal, which is indicative of the vehicle's motion, and a sentiment signal, which is indicative of a sentiment state of the vehicle's driver. The level selector is configured to select a level of obtrusiveness of a warning signal based at least in part on the sentiment signal. The escalator receives the level from the level selectors and outputs a warning signal based on the level.

As used herein, "sentiment" and "emotion" are to be considered to have the same meaning.

In some embodiments, the apparatus further includes a camera, a GPS that maintains a library of constraints that are keyed to vehicle location and that provides a first constraint signal based on the vehicle's location, user preferences that control operation of the warning system, a motion sensor that provides, to the warning system, indicative of the vehicle's speed to the level selector, and a sentiment analyzer that provides, to the level selector, a signal indicative whether the driver is in a neutral state or in a non-neutral state. In such embodiments, the warning system further includes a feature extractor that extracts constraint information from one or more images provided by the camera and a multiplexer that receives a second constraint signal from the feature extractor and a first constraint signal from the GPS and that chooses which of the first and second constraint signals to provide to the level selector.

Also among the embodiments are those that include a sentiment analyzer that produces the sentiment signal for the level selector. Such a sentiment analyzer receives inputs from a camera that is positioned to acquire an image of the driver and a microphone that is positioned to listen to the driver. Among the embodiments that include a sentiment analyzer are those in which the sentiment analyzer produces a signal that is indicative of an extent to which the driver has deviated from a neutral sentiment.

Still other embodiments include user preferences that enable the driver to control and/or configure various aspects of the system, such as to selectively disable a sentiment analyzer, the warning system, or both. Embodiments also include those in which the user preferences configure the warning system to output the warning signal only if a time spent in non-compliant driving exceeds a threshold.

Still other embodiments include an external devices, such as an external camera or a GPS. In those embodiments that feature an external camera, the the warning system includes a feature extractor that receives an image from the external camera and determines the constraint signal based on the image. In those embodiments the include the GPS, the GPS that maintains a library of constraints for specific locations, uses the vehicle's location to choose a constraint from the library, and provides the constraint signal that communicates the constraint to the warning system.

Still other embodiments include a multiplexer that selects the constraint signal from among a plurality of sources of constraint signals.

Embodiments include those in which the level selector is further configured to select the level of obtrusiveness based on the constraint and those in which the level selector is further configured to select the level of obtrusiveness based on an observed pattern in the driver's lack of compliance with one or more constraint, the pattern including two or more instances of lack of compliance with the one or more constraints.

These and other features of the invention will be apparent from the following detailed description and the accompanying figures, in which:

### Description of Drawings

FIG. 1 shows a vehicle having a warning system incorporated therein,
FIG. 2 shows a road to be traversed by the vehicle shown in FIG. 1, and
FIG. 3 shows details of a particular embodiment of the warning system shown in FIG. 1.

### Detailed Description

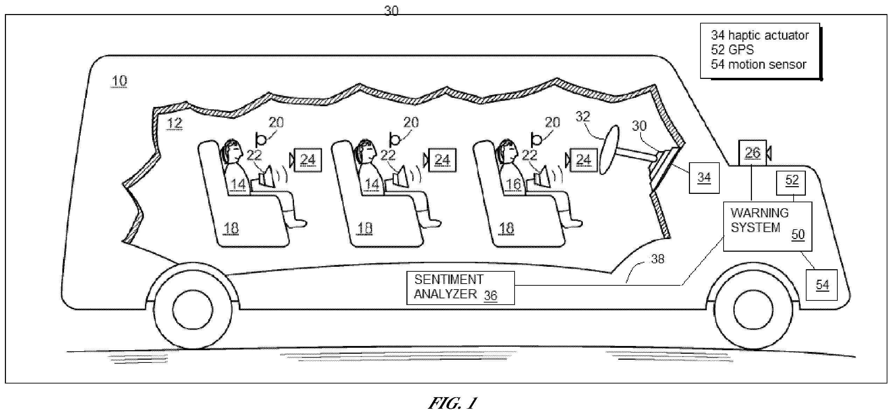

FIG. 1 shows a vehicle 10 having a passenger cabin 12 in which passengers 14 and a driver 16 sit in seats 18. Each seat 18 has an associated microphone 20, a speaker 22, and an internal camera 24. The internal cameras 24 are directed towards various portions of the cabin 12. The vehicle 10 also includes external cameras 26. The external cameras 26 are directed towards the vehicle's environment.

The driver 16 faces an instrument panel 28 having a speedometer 30 integrated therein. In addition, the driver 16 has a steering wheel 32 for use in controlling the vehicle's direction. A haptic actuator 34 couples to one or more structures that, when vibrated or otherwise made to move, will attract the driver's attention. In the illustrated embodiment, the haptic actuator 34 couples to the steering wheel 32.

The vehicle 10 further includes a sentiment analyzer 36 that receives inputs from the particular internal camera 24 and microphone 20 that are best situated to observe the driver 16. Based on features present in those inputs, the sentiment analyzer 36 outputs a sentiment signal 38. This sentiment signal 38 indicates whether the driver's sentiment is in a neutral state or a non-neutral state. In some embodiments, the sentiment signal 38 indicates an extent to which the driver's sentiment deviates from the neutral state.

In some embodiments, the sentiment analyzer 36 also receives inputs from internal cameras 24 that face the passengers 14. In such embodiments, the sentiment signal 38 further includes information indicative of whether any one or more of the passengers 14 is a non-neutral sentiment state. Such information is useful for assessing a driver's potential level of distraction.

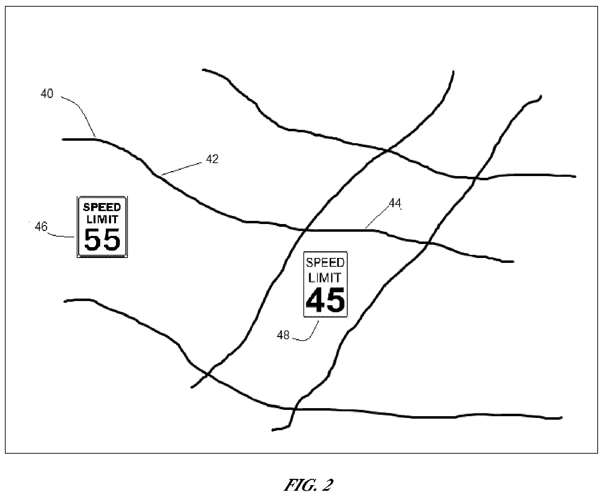

Referring to FIG. 2, a typical roadway 40 along which a vehicle 10 operates includes a first segment 42 and a second segment 44. A first constraint 46 imposes limits on the vehicle's motion within the first segment 42. Similarly, a second constraint 48 imposes limits on the vehicle's motion in the second segment 44.

A typical constraint 46, 48 is a maximum speed. However, certain roadways 40 also specify minimum speeds for vehicles 10 operating thereon. Thus, further examples of constraints 46, 48 include a minimum speed and a band of speeds between a minimum and maximum speed.

Still other examples of constraints 46, 48 include those on the vehicle's direction of travel. For example, a vehicle 10 proceeding in the wrong direction on a one-way street may be traveling below the speed limit but still not in compliance with a constraint 46, 48.

Yet other examples include a change in the vehicle's direction. For example, certain roadways 40, particularly in hilly areas, have "no passing" zones. In such cases, a constraint 46, 48 would permit changes in the vehicle's direction as needed to follow the roadway 40 but not changes that would result in changing lanes. Still another example, of constraints 46, 48 include determining when the vehicle is weaving or is departing from its lane.

It is preferable that the driver 16 operate the vehicle 10 in a manner that complies with the various constraints 46, 48. Referring back to FIG. 1, a warning system 50 assists the user in operating within the constraint 46, 48 corresponding to the particular segment 42, 44. It does so by providing a suitable warning signal 64.

The warning system 50 relies on information concerning the applicable constraint 46, 48. In some embodiments, the warning system 50 uses input from the external camera 26 to identify signage from which it then extracts information concerning the relevant constraint 46, 48. In other embodiments, a GPS 52 maintains a library of constraints 46, 48 for specific locations and uses the vehicle's location to choose the relevant constraint 46, 48, which it then provides to the warning system 50.

The warning system 50 also relies on a motion sensor 54 that provides it with a motion signal 56. The motion signal 56 carries information concerning the vehicle's motion. In some embodiments, the motion signal 56 provides information on the vehicle's speed.

The warning system 50 is further configured to recognize circumstances that require different levels of warning. In particular, the warning system 50 receives the sentiment signal 38 from the sentiment analyzer 36 and chooses an escalated warning level upon recognizing that the driver 16 is in a non-neutral state.

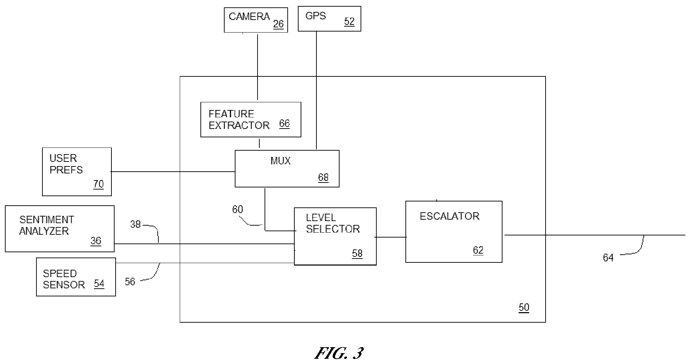

Referring now to FIG. 3, the warning system 50 features a level selector 58 that receives the sentiment signal 38, the motion signal 56. In addition, the level selector 58 receives a constraint signal 60. The constraint signal 60 indicates the constraint for the section of roadway that the vehicle 10 is driving on. Based on these three inputs, the level selector 58 causes an escalator 62 to output different levels of the warning signal 64.

In some embodiments, the level of escalation depends at least in part on the extent of the driver's lack of compliance with constraints. In such embodiments, driving at speeds that are only slightly above the speed limit will trigger only a small escalation whereas driving at speeds that are considerably in excess of the speed limit will trigger a larger escalation.

Similarly, the nature of the driver's lack of compliance is also relevant to the level of escalation. As an example, the danger that arises from driving in the wrong direction is significantly greater than that of driving over the speed limit. In such cases, it is useful for the escalator 62 to immediately escalate the warning signal 64 to its most obtrusive level.

In some embodiments, the warning system 50 relies on observed patterns in the driver's lack of compliance. In such embodiments, the warning system 50 observes a sequence of rapid lane changes or swerving indicates that the driver may also be impaired in some way. When combined with inferences made by the sentiment analyzer 36 based on one or more internal cameras 24 and microphones 20, the warning system 50 is able infer whether the observed pattern has arisen from the driver's intoxication, from distraction by passengers 14, or from road rage. This, in turn, will inform the level of escalation.

As an example, a driver 16 whose judgment is clouded by the effects of road rage will generally not respond well to a stentorian announcement by the warning system 50. Thus, upon inferring, based in part on the sentiment signal 38, that road rage is a likely cause of the driver's lack of compliance, the warning system 50 switches to a calming voice that speaks with prosody suitable to the situation.

In some embodiments, a warning matrix stores information indicating the properties of each levels of the warning signal 64. Each row of the warning matrix maps to a particular warning level. For each row, the columns specify the properties of the warning signal 64 at that level. The level selector 58 sets a pointer to a particular row selected is a function of a three-element vector that includes, as elements thereof, information from the constraint signal 60, the motion signal 56, and the sentiment signal 38.

The different rows are characterized by different levels of obtrusiveness. The movement of a pointer to a more obtrusive warning signal 64 is referred to as an "escalation." The movement of a pointer to a less obtrusive warning signal 64 is referred to as "de-escalation." As used herein, "obtrusiveness" is a measure of the probability that a driver of ordinary skill in driving would notice the warning signal 64.

In some cases, the level selector 58 determines that the vehicle's motion complies with the constraint 46, 48. In such cases, the level selector 58 provides the escalator 62 with a pointer that causes the escalator 62 to output a null warning signal 64.

A null warning signal 64 is one in which nothing is observable by the driver 16. Accordingly, it is indistinguishable from having no warning signal 64 at all. When the pointer points to the null warning signal 64, only escalation is possible.

In other cases, the level selector 58 determines that the vehicle's motion fails to comply with the constraint 46, 48. In such cases, the sentiment signal 38 comes into play.

If the sentiment signal 38 indicates that the driver's sentiment is neutral, the level selector 58 causes the escalator 62 to escalate the warning signal 64 to a first level of obtrusiveness. An example of a warning signal 64 at this first level is able a light on the instrument panel 28 similar to that which typically appears when a vehicle 10 is low on fuel.

If, instead, the sentiment signal 38 indicates that the driver's sentiment deviates from neutral, the level selector 58 causes the escalator 62 to escalate the warning signal 64 to a second level of obtrusiveness. This second level is more obtrusive than the first level.

An example of a warning signal 64 at this second level is a light on the instrument panel in combination with an audible alert, such as a spoken utterance announcing to the driver 16, and to all passengers 14 who may be in the cabin 12, that the driver 16 is currently operating the vehicle 10 in a non-compliant manner. Other examples include those in which the warning signal 64 incorporates a haptic signal. An example of such a haptic signal is one in which the haptic actuator 34 vibrates the steering wheel 32.

In the illustrated embodiment, there are two methods for obtaining the constraint signal 60. The first method comprises receiving the constraint 46, 48 directly from the GPS 52. The second method is to use a feature extractor 66 to receive a signal from the external camera 26 and to extract the relevant constraint 46, 48 from that signal.

Both the signal from the GPS 52 and the signal from the feature extractor 66 are provided to a multiplexer 68. The choice of which to use is made by stored user preferences 70 that are set by a user. In other embodiments, only one or the other method is available, in which case no multiplexer 68 is necessary.

The user preferences 70 also specify other operational details of the warning system 50. For example, by appropriately setting the user preferences 70, it is possible to disable the warning system 50 entirely or to disable the use of the sentiment analyzer 36 to implement the warning system 50. Other settings include the amount of time non-compliant driving is tolerated before a change in the warning signal 64. This is a useful feature to prevent jitter. Such jitter arises when the vehicle 10 is being driven at an average speed that is equal to the speed limit but with minor variances from that average.

Having described the invention and a preferred embodiment thereof, what is claimed as new and secured by letters patent is:

## Claims

1. A method comprising determining that a vehicle is being operated in a manner that fails to comply with a constraint imposed on motion of vehicles on a section of a road, determining that a driver of said vehicle exhibits a non-neutral sentiment, based on having determined that said driver exhibits a non-neutral sentiment, selecting a level of obtrusiveness for said warning message, and outputting said warning message at said level.

2. The method of claim 1, wherein said constraint is a speed limit.

3. The method of claim 1, wherein said level is selected based on an extent to which said non-neutral sentiment deviates from a neutral sentiment.

4. The method of claim 1, further comprising determining said constraint based at least in part on a GPS signal.

5. An apparatus comprising a warning system (**50**) for a vehicle, said warning system comprising an escalator (**62**) and a level selector (**58**), wherein said level selector receives a constraint signal (**60**), a motion signal (**38**), and a sentiment signal (**38**), wherein said constraint signal is indicative on a constraint on motion of a vehicle on a section of a road, wherein said motion signal is indicative of motion of said vehicle, wherein said sentiment signal is indicative of whether a driver of said vehicle has a non-neutral sentiment, wherein said level selector is configured to select a level of obtrusiveness of a warning signal (**64**) based at least in part on said sentiment signal, and wherein said escalator receives said level from said level selectors and outputs a warning signal based on said level.

6. The apparatus of claim 5, further comprising a camera, a GPS that maintains a library of constraints that are keyed to vehicle location and that provides a first constraint signal based on said vehicle's location, user preferences that control operation of said warning system, a motion sensor that provides, to said warning system, indicative of said vehicle's speed to said level selector, and a sentiment analyzer that provides, to said level selector, a signal indicative whether said driver is in a neutral state or in a non-neutral state, wherein said warning system further comprises a feature extractor that extracts constraint information from one or more images provided by said camera and a multiplexer that receives a second constraint signal from said feature extractor and a first constraint signal from said GPS and that chooses which of said first and second constraint signals to provide to said level selector.

7. The apparatus of claim 5, further comprising user preferences that configure said warning system to output said warning signal only if a time spent in non-compliant driving exceeds a threshold.

8. The apparatus of claim 5, further comprising an external camera, wherein said warning system comprises a feature extractor that receives an image from said external camera and determines said constraint signal based on said image.

9. The apparatus of claim 5, further comprising a GPS that maintains a library of constraints for specific locations, uses said vehicle's location to choose a constraint from said library, and provides said constraint signal that communicates said constraint to said warning system.

10. The apparatus of claim 5, wherein said warning system further comprises a multiplexer that selects said constraint signal from among a plurality of sources of constraint signals.

11. The apparatus of claim 5, further comprising a sentiment analyzer that produces a signal that is indicative of an extent to which said driver has deviated from a neutral sentiment.

12. The apparatus of claim 5, wherein said level selector is further configured to select said level of obtrusiveness based on said constraint.

13. The apparatus of claim 5, wherein said level selector is further configured to select said level of obtrusiveness based on an observed pattern in said driver's lack of compliance with said constraint, said pattern comprising two or more instances of lack of compliance with said constraint.

14. The apparatus of claim 5, further comprising user preferences to enable said driver to selectively disable said warning system.

15. The apparatus of claim 5, wherein said level selector is further configured to select said level of obtrusiveness based on said constraint.
